# EUROPÄISCHE PATENTSCHRIFT

(11) **EP 2 814 451 B1**
(45) Veröffentlichungstag und Bekanntmachung des Hinweises auf die Patenterteilung: **18.08.2021**
(21) Anmeldenummer: 13703602.6
(22) Anmeldetag: 12.02.2013
(51) Int. Cl.: A61K 8/06, A61K 8/37, A61Q 19/00

(54) **STABILE WASSER IN ÖL EMULSIONEN MIT SPREITFÄHIGEN ÖLEN**
STABLE WATER-IN-OIL EMULSIONS WITH SPREADABLE OILS
ÉMULSIONS EAU DANS L'HUILE STABLES, À L'AIDE D'HUILES POUVANT ÊTRE ÉTALÉES

(30) Priorität: 16.02.2012 DE 102012002951
(43) Veröffentlichungstag der Anmeldung: 24.12.2014
(60) Teilanmeldung: 20173203.9
(73) Patentinhaber: Beiersdorf AG, 20253 Hamburg (DE)
(72) Erfinder: VON DER FECHT, Stephanie, 22880 Wedel (DE); MÖLLGAARD, Svenja, Lena, 22337 Hamburg (DE); Koch, Petra, 20146 Hamburg (DE); BALCKE, Isabel, 22337 Hamburg (DE)
(74) Vertreter: Beiersdorf AG
(86) Internationale Anmeldenummer: PCT/EP2013/052741
(87) Internationale Veröffentlichungsnummer: WO 2013/120829

(56) Entgegenhaltungen:
- EP-A2- 1 147 760
- EP-A2- 2 123 257
- WO-A1-2010/125541
- DE-A1- 10 361 568
- FR-A1- 2 927 535
- DATABASE GNPD [Online] MINTEL; 31. Januar 2012 (2012-01-31), Migros: "Zoe Cream & Oil Body Milk", XP002716995, Database accession no. 1709361
- DATABASE GNPD [Online] MINTEL; 30. November 2010 (2010-11-30), Mibelle: "I Am Body - Regenarative Body Milk", XP002716996, Database accession no. 1428287
- MEYER J ET AL: "A novel PEG-free emulsifier designed for formulating W/O lotions with a light skin feel", SOFW-JOURNAL SEIFEN, OELE, FETTE, WACHSE, VERLAG FUR CHEMISCHE INDUSTRIE, AUGSBURG, DE, Bd. 131, Nr. 11, 1. Januar 2005 (2005-01-01), Seiten 20-28, XP009104229, ISSN: 0942-7694
- None

## Beschreibung

Die Erfindung ist in den Ansprüchen definiert und umfasst eine kosmetische und/oder dermatologische Zubereitung auf Basis einer Wasser in Öl Emulsion umfassend mindestens zwei W/O-Emulgatoren und mindestens ein Öl mit einem Spreitwert größer 600 mm²/10min.

Als kosmetische oder medizinische Zubereitungen sind oftmals Emulsionen, insbesondere W/O-, O/W-, O/W/O oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.
Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.
Der Stand der Technik kennt mehrere wesentliche Faktoren, die einen positiven Einfluss auf die Stabilität und Rheologie von Emulsionen haben.
Emulsionen benötigen zu ihrer Bildung und zur Stabilisierung im Allgemeinen einen oder mehrere Emulgatoren, Verdicker und/oder Konsistenzgeber um über einen kosmetisch akzeptablen Zeitraum stabil zu sein, im Allgemeinen 1 Jahr nach dem Öffnen einer kosmetischen Zubereitung.
Eine besondere Herausforderung stellt die Formulierung von fließfähigen Emulsionen dar. Diese werden aufgrund ihrer ansprechenden Verteilbarkeit vom Verbraucher sehr geschätzt, jedoch ist die stabile Formulierung eine technologische Herausforderung.
Zur Stabilisierung von Emulsionen werden häufig ethoxilierte Emulgatoren verwendet, die, allgemein bekannt, zu belastbaren, stabilen Emulsionszubereitungen führen und oft einen relativ breiten sensorischen Bereich abdecken können. Bekannt ist jedoch, dass ethoxilierte Emulgatoren durch ihre PEG-Einheiten als Penetrationsenhancer wirken.

So offenbart EP 1 192 935 A2 W/O-Emulsionen umfassend Polyether wie PEG-45 (dodecyl glycol) copolymer und PEG-22 (dodecyl glycol) copolymer.

Die Verwendung von Polyethylenglykolen und/oder Polyethylenglykolderivaten und deren Abkömmlingen werden in der Öffentlichkeit kontrovers diskutiert, da sie im Verdacht stehen nach topische Applikation die Haut durchlässiger für Fremdkörper wie z.B. Schadstoffe zu machen.
Weiterhin können unter Einwirkung von Sonnenstrahlung die photoinstabilen Polyethylenglykolhaltigen (PEG)-Emulgatoren zersetzt werden und unschöne Hautreaktionen auslösen.
Aus den genannten Gründen werden vom Verbraucher zunehmend kosmetische Formulierungen gesucht, die frei von dieser Substanzklasse sind.
Auch um W/O-Emulsionen sensorisch ansprechend entwickeln zu können, werden derzeit meist PEG-Emulgatoren verwendet. Da viele Verbraucher Produkte suchen, die frei von dieser Substanz-Klasse sind, ist es eine technologische Herausforderung W/O-Emulsionen ohne diese Stoffe zu stabilisieren und gleichzeitig ein ansprechendes Hautgefühl zu erlangen.
Hinsichtlich der Stabilität ist die Herausforderung insbesondere das scaling-up und beim Hautgefühl sind Parameter wie "schnelles und leichtes Einziehen, geringe Klebrigkeit" schwer ohne PEG-Stabilisatoren zu erreichen

Wünschenswert ist es daher Emulsionszubereitung ohne ethoxilierte Emulgatoren zur Verfügung zu stellen, die aber dennoch möglichst breit variierbar und vor allem stabile Emulsionen darstellen.
Weiterhin müssen kosmetische oder dermatologische Zubereitungen einigen ästhetischen und sensorischen Gesichtspunkten genügen um eine ausreichende Verbraucherakzeptanz zu erlangen.

Bekannt ist aber, dass der Zusatz an Hautbefeuchtungsmitteln häufig dazu führt, dass die Zubereitungen klebrig wirken.

Für W/O-Emulsionen ist bekannt, dass die Viskosität abhängig von der eingesetzten Emulgatormenge ist. Wird eine hohe Konzentration eingesetzt, werden Cremes mit einer hohen Konsistenz erhalten, bei Reduktion der Einsatzkonzentration sind die Systeme zwar fließfähig, oft aber nicht mehr stabil, dies zeigt sich insbesondere im up-scaling. Somit stellt die Formulierung von fließfähigen W/O-Emulsionen derzeit eine große technologische Herausforderung dar.
Die Viskosität ist ein Maß für die Zähflüssigkeit eines Fluids. Der Kehrwert der Viskosität ist die Fluidität, ein Maß für die Fließfähigkeit eines Fluids. Je größer die Viskosität, desto dickflüssiger (weniger fließfähig) ist das Fluid; je niedriger die Viskosität, desto dünnflüssiger (fließfähiger) ist es. Die Angabe von Viskositäts-, Fließfähigkeitswerten bezieht sich dabei häufig auf Raumtempeatur (20°C).

WO 2009080657 A2 offenbart W/O-Emulsionen umfassend neben hydrophob modifizierten Polysacchariden, Stärken und/oder Agar Polyglycerol-4 Diisostearat Polyhydroxystearat Sebacate (Isolan GPS ®).
FR 2927535 offenbart stabile Wasser in Öl Emulsionen als kosmetische Zubereitungen umfassend Ester der Fettsäuren und Polyole. Als bevorzugter Ester ist u.a. Polyglycerol-4 Diisostearat Polyhydroxystearat Sebacate (Isolan GPS ®) genannt. Glycerin wird bevorzugt in den beschriebenen Zubereitungen gewählt. Die Zubereitungen sollen nicht klebrig auf der Haut sein.
WO 2008/055692 A2 beschreibt silikonfreie Hautschutzmittel umfassend neben Ölen und Polyolen u.a. als Emulgatoren Polyglycerol-4 Diisostearat Polyhydroxystearat Sebacate und/oder Polyglyceryl-2 Dipolyhydroxystearate.

DE 60 2004 013 358 T2 offenbart mehrphasige Emulsionen. Bei den beschriebenen Emulsionen handelt es sich um "Mehrfach-Wasser-in-Öl-in-Wasser-Emulsionen" d.h. W/O/W-Systeme. Darüber hinaus wird als W/O-Emulgator lediglich Polyglyceryl-3 Diisostearat genannt.

DE 10 2008 028 822 A1 offenbart ein umfangreiches Naschlagewerk zu Emulgatoren und Ölen. Konkret werden kosmetische Stiftzusammensetzungen in Form von Öl in Wasser Dispersionen/Emulsionen beschrieben. Stiftzusammensetzungen sind bei Raumtemperatur nicht als fließfähig zu bezeichnen.

DE 199 24 277 A1 beschreibt W/O-Emulsionen und ihre bekannten Vor- und Nachteile. Die Nachteile sollen durch den Einsatz von grenzflächenaktiven Substanzen aus der Gruppe der Alkylmethiconcoplyole beseitigt werden.

Eine für den Verbraucher sehr wesentliche, dabei aber nur schwer quantitativ messbare Eigenschaft kosmetischer Produkte ist ihre Textur und Sensorik. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- Visuelle Eindrücke: alle mit dem Auge wahrnehmbaren Merkmale (Farbe, Form, Struktur).
- Olfaktorische Eindrücke: alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- Haptische Eindrücke: alle Empfindungen des Tastsinns, die vornehmlich Gefüge und Konsistenz des Produktes betreffen.
Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.
Eine weitere Aufgabe der vorliegenden Erfindung war es daher, Zubereitungen zu Verfügung zu stellen, welche neben den für Kosmetika üblichen Kriterien wie Verträglichkeit, Lagerstabilität und dergleichen auch für den Verbraucher wesentliche, bisher nicht gekannte kosmetische, insbesondere sensorische, Leistungen bieten. Insbesondere war es ein Ziel eine lagerstabile Formulierung zu schaffen, die Pflege vermittelt, ohne dabei glitschig, ölig oder fettig zu sein und gleichzeitig eine eindeutige "Lotion-Anmutung", d.h. fließfähig, und KEINE Creme-Anmutung zeigt.
Die Erfindung ist eine kosmetische und/oder dermatologische fließfähige Zubereitung auf Basis einer Wasser in Öl Emulsion umfassend mindestens zwei W/O-Emulgatoren und mindestens ein Öl mit einem Spreitwert größer 600 mm²/10min.
Die Fließfähigkeit der Zubereitung bedeutet, dass die Zubereitung bei Raumtemperatur (20°C) fließfähig ist.

Als W/O-Emulgatoren werden Diisostearoyl Polyglyceryl-3 Dimer Dilinoleat und Polyglyceryl-4 Diisostearte/Polyhydroxystearat/Sebacate gewählt.
Diisostearoyl Polyglyceryl-3 Dimer Dilinoleate sind PEG-freie Emulgatoren und als Isolan® PDI und Polyglyceryl-4 Diisostearate/Polyhydroxystearate/Sebacate als Isolan® GPS der Fa.
Evonik mit einem HLB-Wert von ca. 5 bekannt
Überraschender Weise ist es durch den Einsatz zweier erfindungsgemäßer W/O-Emulgatoren in Kombination mit dem/den leicht spreizenden Ölen gelungen, die genannten technischen Herausforderungen zu lösen.
Die Verwendung einer Kombination aus zwei W/O-Emulgatoren in Kombination mit einem oder mehreren leicht spreizenden Ölen führt zu fließfähigen, leicht verteilbaren Lotionen mit einer ansprechenden, d.h. nicht öligen Sensorik.
Bevorzugt werden nur zwei erfindungsgemäße W/O-Emulgatoren eingesetzt, der Anteil weiterer Emulgatoren liegt dann bei 0 Gew.%.

Die beiden erfindungsgemäß eingesetzen Emulgatoren sind Diisostearoyl Polyglyceryl-3 Dimer Dilinoleat und Polyglyceryl-4

Diisostearate/Polyhydroxystearate/Sebacat. Durch den Einsatz relativ hoher Mengen an leicht spreitenden Ölen wird die Sensorik der erfindungsgemäßen Zubereitung erstaunlicherweise optimiert. Leicht spreitende Öle sind dabei Öle mit einer Spreitfähigkeit von größer als 600 (qmm/10min) und bevorzugt einer Viskosität von kleiner als 10 mPas. Besonders bevorzugt ist dabei Isopropylpalmitat.
In einem Vergleichsversuch wurden die Viskositäten zweier W/O Emulsionszubereitungen untersucht, wie in nachfolgender Tabelle angegeben.

| | Viskosität in mPas | Anmutung und Sensorik |
|---|---|---|
| Beispiel-Rezeptur 4 | 5400 | Fließfähige Lotion mit angenehmem Hautgefühl, pflegend aber nicht ölig oder fettig |
| Beispiel-Rezeptur 4 ohne Isopropylpalmitate dafür mit 9,5% Tri Isostearin | 26 750 | Reichhaltige Creme, fettig und ölig |
| Spreitfähigkeit Isopropylpalmitate: 625 (mm²/10min) | | |
| Spreitfähigkeit Tri Isostearin: 310 (mm²/10min) | | |

Die W/O Emulsionszubereitung ohne erfindungsgemäße Öle zeigte eine Viskosität von 26750 mPas und war eine reichhaltige Creme. Lediglich durch den Austausch von einem Öl mit einem geringen Spreitwert (Tri Isostearin) gegen ein Öl mit einem hohen Spreitwert (Isopropyl Palmitat) konnte die Viskosität der Zubereitung auf 5400 mPas reduziert werden, so dass als Produkt eine Lotion mit angenehmem Hautgefühl erhalten wurde. Überraschend ist, dass lediglich durch den Austausch eines Öles die Viskosität der Zubereitung dermaßen grundlegend beeinflusst wurde.

Ein oder mehrere Öle mit einem Spreitwert größer 600 mm²/10min sind daher erstaunlicherweise zur Veränderung, insbesondere Verringerung der Viskosität der sie enthaltenen kosmetischen Wasser in Öl Emulsionen zu verwenden.
Durch Austausch der Ölphase durch Öle mit einem Spreitwert größer 600 mm²/10min lassen sich Zubereitungen bereit stellen, die sich den Verbraucherwünschen individuell anpassen lassen.

Ziel der Erfindung war es eine lagerstabile Formulierung zu schaffen, die Pflege vermittelt, ohne dabei glitschig, ölig oder fettig zu sein. Das ist genau mit der erfindungsgemäßen Formulierung gelungen. Sie hat mehr Struktur und vermittelt dies auch durch den senorischen Eindruck. Der Verbraucher übersetzt dies gerne in "vermittelt Pflege, ohne unangenehme Nebenwirkungen wie Öligkeit oder Fettigkeit zu zeigen".
Besonderheit ist, dass die Formulierung trotzdem fließfähig, also als Lotion anwendbar ist. Lotions-Anmutung, d.h. fließfähig sind dabei vorteilhaft Formulierungen, die eine Viskosität von größer 4000 mPas (20°C) und den sensorischen "Creme"-Effekt nicht aufweisen.

Überraschend dabei ist auch, dass dies nicht auf Kosten der Lagerstabilität erfolgt. Der Kosmetikfachmann hat nun eine Möglichkeit in der Hand individuelle Verbraucherwünsche nach verbesserter Sensorik zu erfüllen ohne dabei befürchten zu müssen, die Sicherheit und Stabilität zu vernachlässigen.

Bevorzugt umfassen die erfindungsgemäßen Zubereitungen keine weiteren zusätzlichen Emulgatoren. Der Anteil an zusätzlichen Emulgatoren sollte somit bevorzugt unter 0,01 Gew.% liegen, bezogen auf die Gesamtmasse der Zubereitung, um als erfindungsgemäß - ohne zusätzlichen Emulgator - zu gelten.

In gleicher Weise kann erfindungsgemäß auf den Zusatz an Polyethylenglykolen und/oder Polyethylenglykolderivaten verzichtet werden. Der Anteil an PEGs liegt daher unter 1 Gew.%, insbesondere 0 Gew.%, bezogen auf die Gesamtmasse der Zubereitung.

Der Ölanteil der erfindungsgemäßen Zubereitungen liegt vorteilhaft im Bereich von 10 bis 30 Gew.%, bevorzugt zwischen 15 bis 25 Gew.%, besonders bevorzugt im Bereich von 18 bis 22 Gew., bezogen auf die Gesamtmasse der Zubereitung.
Die Lipidphase umfasst dabei erfindungsgemäß mindestens ein Öl mit einem Spreitwert größer 600 (qmm/10min). Bevorzugt ist wenn zusätzlich ein zweites Öl mit einem Spreitwert größer 600 (qmm/10min), bevorzugt größer 800 mm²/10 Min., besonders bevorzugt größer 1000 mm2/10 Min enthalten ist.
Bevorzugt sind ein oder mehrere der in der nachfolgenden Tabelle 1 aufgeführten Öle mit einem Spreitwert über 600 mm²/10 Min. und einer Viskosität kleiner 10 mPas auszuwählen.

**Tabelle 1: Öle mit einem Spreitwert größer 600 mm²/10 min**

| INCI | Viskosität [mPas] | Spreitfähigkeit (20 µl/Rotbandfilter) [mm2/10 min] |
|---|---|---|
| Isopropyl Palmitate | 6,2 | 625 |
| Isopropyl Myristate | 4,6 | 707 |
| Coco-Capryl ate/Caprate | 5 | 755 |
| Isopropyl Isostearate | 9,5 | 790 |
| Cyclomethicone | 4 - 5 | 804 - 845 |
| Butylene Glycol Dicaprylate/Dicaprate | 9,6 | 813 |
| Dicaprylyl Carbonate | 6,3 | 875 |
| Isopropyl Stearate | 6,8 | 910 |
| Ethylhexyl Cocoate | 7,9 | 930 |
| Dibutyl Adipate | 5 | 935 |
| Isodecyl Neopentanoate | 3,8 | 962 |
| Isohexadecane | 3,7 | 990 |
| C13-16 Isoparaffin | 2,6 | 1018 |
| Dicaprylyl Ether | 3,3 | 1020 |

Als bevorzugte Öle sind Isopropylpalmitat mit einem Spreitwert von ca. 625 mm²/10 Min. und C13-16 Isoparaffin mit einem Spreitwert ca. 1018 mm²/10 Min zu verwenden.
Bezogen auf die gesamte Zubereitung liegt der Anteil an Ölen mit einem Spreitwert größer 600 mm²/10 Min. bei 10 bis 20 Gew.%, bezogen auf die Gesamtmasse der Zubereitung. Bezogen auf die Gesamtmasse an Ölphase liegt der Anteil an Ölen mit einem Spreitwert größer 600 mm²/10 Min. vorteilhaft bei 30 bis 90 Gew.%, bevorzugt bei 40 bis 80, besonders bevorzugt bei 40 bis 60 Gew.%, bezogen auf die Gesamtmasse der Ölphase.

Überraschender Weise ist es durch den Einsatz der leicht spreitbaren Öle gelungen, stabile, fließfähige (bei RT) W/O-Emulsionen zu formulieren.
Bevorzugt sind Zubereitungen auf Basis einer Wasser in Öl Emulsion umfassedn ein Öl mit einem Spreitwert größer 600 mm²/10min. und ein Öl mit einem Spreitwert über 800 mm²/10min, insbesondere vorteilhaft ist es, wenn das 2. spreitbare Öle einen Spreitwert über 1000 mm²/10min. aufweist.
Vorteilhaft waren dabei Öle mit einem Spreitwert größer 600 qmm/10min und einer Viskosität kleiner als 10 mPas, insbesondere vorteilhaft mit einem Spreitwert zwischen 1020 und 600, namentlich Isopropylpalmitat und/oder C13-16 Isoparaffin.

Bei 25°C weist Isopropylpalmitat eine Viskosität von 6,2 mPas auf, gemessen mit Hilfe eines Viscotester VT-02, der Firma Haake bei 25°C mit Hilfe des Drehkörpers 1 bzw. 2 und Ablesung der Skala 1 bzw. 2 bestimmt, entsprechend einer Scherrate von 10 Pa/s.
Die Fähigkeit sich wenig oder vermehrt gut auszubreiten auf der Haut wird in der Kosmetik als Spreitfähigkeit bezeichnet. Je höher die Spreitfähigkeit eines Öles ist, umso leichter verteilt es sich auf der menschlichen Haut.
Die Messung des Spreitwertes (in mm²/10 min) erfolgt vorteilhaft nach folgendem Verfahren: 20 µl der zu untersuchenden Substanz werden auf ein Rotband-Filterpapier der Fa. Schleicher & Schüll mittig getropft. Sogleich wird eine Stoppuhr gestartet und nach 10 Minuten die Fläche gemessen, die in dieser Zeit von der Substanz benetzt wurde.
Die Messung wird in einem temperaturkostantem Raum bei 25°C +/- 1°C durchgeführt.

Hydrophile Stabilisatoren wie Verdicker und Fettalkohole können erfindungsgemäß optional enthalten sein.

Die erfindungsgemäßen Zubereitungen umfassen vorteilhaft mindestens ein Hautbefeuchtungsmittel zu einem Anteil von insgesamt 5 bis 20 Gew.%, bezogen auf die Gesamtmasse der Zubereitung. Bevorzugt ist ein Glycerin-Gehalt von 7 bis 15 Gew.% beonders bevorzugt von 10 Gew.%.

Durch den Einsatz der erfindungsgemäßen Emulgatorkombination ist es gelungen, eine W/O-Emulsion zu formulieren, die trotz hoher Mengen an Hautbefeuchtungsmitteln wie Glycerin ein sehr ansprechendes Hautgefühl aufweist und auch im bei Streßtests wie z.B. Lagerung bei erhöhten bzw. wechslenden Tempertauren stabil ist. Diese Stabilität besteht nicht nur bei den im Labormaßstab hergestellten 1 bis 5 kg-Ansätzen sondern auch bei größeren Mengen wie z.B. 100 bis 500 kg und auch bei der Produktion im Tonnen-Maßstab. Dieser als "up-scaling" bekannte Prozess ist bekanntermaßen für übliche W/O-Emulsionen besonders sensibel. Für die erfindungsgemäßen Zubereitungen stellte sich der up-scaling-Prozess als überraschend problemlos dar.
Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert werden. Emulgatoren haben primär keinen waschaktiven, tensidischen Charakter. Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.
Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).
Es wird dabei unterschieden zwischen nicht-ionisch, anionisch und kationischen Emulgatoren.
Ein Kennzeichen für die Hydrophilie eines gegebenen Emulgators ist dessen HLB-Wert, der sich nach folgender Formel ergibt: HLB = 20 x (1- Mₗᵢₚₒₚₕᵢₗ/M), wobei Mₗᵢₚₒₚₕᵢₗ für die Molmasse des lipophilen Anteils im Emulgator und M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler bezeichnet.

Erfindungsgemäß hat sich nun herausgestellt, dass bei Wahl zweier W/O-Emulgatoren, die bevorzugt zu einem Anteil von mehr als 1,5 Gew.% in der Zubereitung enthalten sind und zusätzlich mindestens ein leicht spreitendes Öl enthalten ist, fließfähige Lotionen erhalten werden, die sich durch eine pflegende aber dabei nicht ölige und nicht fettige Sensorik auszeichnen (siehe Beispielrezepturen). Diese Rezepturen sind zudem über einen langen Zeitraum stabil, erstaunlicherweise auch bei Lagerung bei unterschiedlichen bzw. schwankenden Temperaturen. Bekanntermaßen gibt es bei der Produktion von größeren Produktmengen dem s.g. up-scaling bei solchen Rezepturen oft Probleme. Dieser als "up-scaling" bekannte Prozess ist bekanntermaßen für übliche W/O-Emulsionen besonders sensibel. Für die im Rahmen dieser Erfindung dargestellten Zubereitungen stellte sich der up-scaling-Prozess als überraschend problemlos dar, was sich auf die erfindungsgemäße Emulgatorwahl gründet.
Die erfindungsgemäße kosmetische und/oder dermatologische Zubereitung ist eine bei Raumtemperatur fließfähige Wasser in Öl Emulsion und keine Mehrfachemulsion, wie W/O/W- oder O/W/O-Emulsion. Vorteilhaft ist die erfindungsgemäße Emulsion nicht als Mikro- oder Nanoemulsion formuliert.
Die Erfindung ist bevorzugt eine kosmetische oder dermatologische Zubereitung auf Basis einer Wasser in Öl Emulsion, die keine (0%) ethoxilierte Emulgatoren, Polyethylenglykole und/oder Polyethylenglykolderivate aufweist.

Weiterhin umfassen die erfindungsgemäßen Zubereitungen ein oder mehrere Puderrohstoffe, die bevorzugt zu einem Anteil von bis zu 5 Gew.%, bevorzugt 0,2 bis 2 Gew.%, bezogen auf die Gesamtmasse der Zubereitung enthalten sind. Bevorzugte Puderrohstoffe sind Aluminum Starch Octenylsuccinat und/oder Talkum.
Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc, sofern der Zusatz die geforderten Eigenschaften hinsichtlich PEG-freiheit, Emulgatorgehalt, geforderter Stabilität und Sensorik nicht behindert.

Der Wassergehalt der erfindungsgemäßen Zubereitungen liegt vorteilhaft zwischen 40 und 80 Gew.%, bevorzugt zwischen 50 Gew.% und 70 Gew.%, besonders bevorzugt zwischen 55 Gew.% und 65 Gew.%, jeweils bezogen auf die Gesamtmasse der Zubereitungen.

Bei Einschränkungen auf bevorzugt genannte Stoffe, seien es die Lipide mit erfindungsgemäßen Spreitwert, die W/O Emulgatoren oder weitere bevorzugt gennannte Bestandteile, so beziehen sich deren bevorzugten Anteilsbereiche dann auch auf die dann ausgewählten Einzelbestandteile. Die anderen, die durch die Einschränkung ausgeschlossenen Bestandteile, zählen dann nicht mehr zu den aufgeführten Anteilsbereichen hinzu.

Die nachfolgenden Beispiele illustrieren die erfindungsgemäßen Zubereitungen. Die Zahlenangaben beziehen sich auf die Gewichtsanteile in Bezug zur Gesamtmasse der Zubereitung, sofern nichts anderes angegeben ist.

### Beispiel 1:

0,5 % Polyglyceryl-4 Diisostearate/Polyhydroxystearate/Sebacate
1,9 % Diisostearoyl Polyglyceryl-3 Dimer Dilinoleate
3% Cera Microcristallina
10% Isopropyl Myristate
10% Dicapylyl Ether
1% Talkum
12% Glycerin
1% Propylenglycol
0,1% Hexandiol
0,15% Potassium Sorbate
0,1% Citric Acid
0,2% Sodium Citrate
0,4% Parfüm
Mit Aqua ad 100%

### Beispiel 2:

0,8 % Polyglyceryl-4 Diisostearate/Polyhydroxystearate/Sebacate
1,9 % Diisostearoyl Polyglyceryl-3 Dimer Dilinoleate
2,5 % Cetylpalmitate
0,1% Cera Microcristallina
2% C13-16 Isoparaffin
2% Cyclomethicone
4% Argania Spinosa Kernel Oil
0,1% Calendula Oil
6,5% Paraffinum Liquidum
6,5% Isopropyl Palmitate
10% Glycerin
2% Tapoca Starch
0,15% Potassium Sorbate
0,2% Sodium Citrate
0,1% Citric Acid
0,15% Parfüm
Mit Aqua ad 100%

### Beispiel 3:

1,5 % Polyglyceryl-4 Diisostearate/Polyhydroxystearate/Sebacate
1,5 % Diisostearoyl Polyglyceryl-3 Dimer Dilinoleate
2,7% Shea Butter
7,5% Isohexadecane
5, 3% Dibutyl Adipate
6,5% Paraffinum Liquidum
0,1% Sonnenblumenöl
13,75% Glycerin
0,5% Ethylhexylglycerin
1% Aluminium Starch Octenylsuccinate
1% Nylon-12
0,15% Potassium Sorbate
0,2% Sodium Citrate
0,1% Citric Acid
0,3% Parfüm
Mit Aqua ad 100%

### Beispiel 4:

0,8 % Polyglyceryl-4 Diisostearate/Polyhydroxystearate/Sebacate
1,4 % Diisostearoyl Polyglyceryl-3 Dimer Dilinoleate
1 % Cera Microcristallina + Paraffinum Liquidum
0,5 % Cera Microcristallina
2 % Paraffinum Liquidum
1% Prunus Amygdalus Dulcis Oil
0,5% Cetyl Palmitate
9,5% Isopropylpalmitate
6% C13-16 Isoparaffin
0,5% Aluminium Starch Octenylsuccinate
10% Glycerin
0,15% Potassium Sorbate
0,7 Magnesium Sulfate
0,1% Maris Sal
0,5% Glyceryl Glucoside
0,2% Sodium Citrate
0,1% Citric Acid
0,35% Parfüm
Mit Aqua ad 100%

### Beispiel 5:

1,4 % Polyglyceryl-4 Diisostearate/Polyhydroxystearate/Sebacate
0,8 % Diisostearoyl Polyglyceryl-3 Dimer Dilinoleate
0,2 % Cera Microcristallina
1% Shea Butter
2,5% Cera Microcristallina + Paraffinum Liquidum
7,5% Caprylic/Capric Triglyceride
12 % Dicaprylyl Ether
0,1% Olivenöl
1% Talkum
0,2% Aluminium Starch Octenylsuccinate
12% Glycerin
0,1% Caprylyl Glycol
0,15% Potassium Sorbate
0,1% Citric Acid
0,2% Sodium Citrate
0,4% Parfüm
Mit Aqua ad 100%

### Beispiel 6:

1,1 % Polyglyceryl-4 Diisostearate/Polyhydroxystearate/Sebacate
1,2 % Diisostearoyl Polyglyceryl-3 Dimer Dilinoleate
0,2 % Cera Microcristallina
1% Shea Butter
2,5% Cera Microcristallina + Paraffinum Liquidum
7,5% C13-16 Isoparaffin
12 % Dicaprylyl Ether
0,1% Olivenöl
1% Talkum
0,2% Aluminium Starch Octenylsuccinate
12% Glycerin
0,1% Caprylyl Glycol
0,15% Potassium Sorbate
0,1% Citric Acid
0,2% Sodium Citrate
0,4% Parfüm
Mit Aqua ad 100%

## Patentansprüche

1. Kosmetische und/oder dermatologische bei Raumtemperatur fließfähige Zubereitung auf Basis einer Wasser in Öl Emulsion umfassend
mindestens zwei W/O-Emulgatoren, **dadurch gekennzeichnet, dass** als W/O Emulgatoren Diisostearoyl Polyglyceryl-3 Dimer Dilinoleat und Polyglyceryl-4 Diisostearat/Polyhydroxystearat/Sebacat gewählt werden und
mindestens ein Öl mit einem Spreitwert größer 600 mm²/10min, gewählt aus der Gruppe Isopropyl Palmitat, Isopropyl Myristat, Coco-Caprylate/Caprat, Isopropyl Isostearat, Cyclomethicon, Butylene Glycol Dicaprylat/Dicaprat, Dicaprylyl Carbonat, Isopropyl Stearat, Dibutyl Adipat, Isodecyl Neopentanoat, Isohexadecan, C13-16 Isoparaffin und/oder Dicaprylyl Ether,
wobei der Anteil dieses oder dieser Öle im Bereich 10 bis 20 Gew.%, bezogen auf die Gesamtmasse der Zubereitung, gewählt wird,
wobei zur Messung des Spreitwertes (in mm²/10 min) 20 µl der zu untersuchenden Substanz auf ein Rotband-Filterpapier der Fa. Schleicher & Schüll mittig getropft werden, sogleich eine Stoppuhr gestartet wird und nach 10 Minuten die Fläche gemessen wird, die in dieser Zeit von der Substanz benetzt wurde, wobei die Messung in einem temperaturkostantem Raum bei 25°C +/- 1°C durchgeführt wird,
wobei der Anteil an Polyethylenglykolen und/oder Polyethylenglykolderivaten unter 1 Gew.%, insbesondere 0 Gew.%, bezogen auf die Gesamtmasse der Zubereitung, beträgt und wobei
ein oder mehrere Puderrohstoffe enthalten sind.

2. Zubereitung nach Anspruch 1 **dadurch gekennzeichnet, dass** nur zwei W/O-Emulgatoren enthalten sind.

3. Zubereitung nach einem der vorstehenden Ansprüche **dadurch gekennzeichnet, dass** die Öle mit einem Spreitwert größer 600 mm²/10min eine Viskosität von 10 mPas und weniger aufweisen.

4. Zubereitung nach einem der vorstehenden Ansprüche **dadurch gekennzeichnet, dass** der Anteil an Ölen mit einem Spreitwert größer 600 mm²/10 Min. im Bereich von 30 bis 90 Gew.%, bevorzugt bei 40 bis 80, besonders bevorzugt bei 40 bis 60 Gew.%, bezogen auf die Gesamtmasse der Ölphase, gewählt wird.

5. Zubereitung nach einem der vorstehenden Ansprüche **dadurch gekennzeichnet, dass** die Öle mit einem Spreitwert größer 600 mm²/10 Min. aus der Gruppe Isopropylpalmitat und C13-16 Isoparaffin gewählt werden.

6. Zubereitung nach einem der vorstehenden Ansprüche **dadurch gekennzeichnet, dass** ein Öl mit einem Spreitwert größer 600 mm²/10 Min. und mindestens ein Öl mit einem Spreitwert größer 1000 mm²/10 Min. enthalten sind.

7. Zubereitung nach einem der vorstehenden Ansprüche umfassend mindestens ein Hautbefeuchtungsmittel zu einem Anteil von insgesamt 5 bis 20 Gew.%, bezogen auf die Gesamtmasse der Zubereitung.

8. Zubereitung nach Anspruch 7 **dadurch gekennzeichnet, dass** als Hautbefeuchtungsmittel Glycerin gewählt wird.

9. Zubereitung nach Anspruch 1 **dadurch gekennzeichnet, dass** der Anteil an Puderrohstoffen bis zu 5 Gew.%, insbesondere 0,2 bis 2 Gew.%, bezogen auf die Gesamtmasse der Zubereitung, beträgt.

10. Zubereitung nach Anspruch 1 oder 9 **dadurch gekennzeichnet, dass** als Puderrohstoff Aluminum Starch Octenylsuccinat und/oder Talkum gewählt wird.

11. Zubereitung nach einem der vorstehenden Ansprüche **dadurch gekennzeichnet, dass** der Wasseranteil im Bereich von 40 bis 80 Gew.%, insbesondere zwischen 55 und 65 Gew.%, bezogen auf die Gesamtmasse der Zubereitung, gewählt wird.

## Claims

1. Cosmetic and/or dermatological preparation, flowable at room temperature, based on a water-in-oil emulsion comprising
at least two W/O emulsifiers, **characterized in that** the W/O emulsifiers selected are diisostearoyl polyglyceryl-3 dimer dilinoleate and polyglyceryl-4 diisostearate/polyhydroxystearate/sebacate and
at least one oil with a spreadability value greater than 600 mm²/10 min, selected from the group comprising isopropyl palmitate, isopropyl myristate, coco-caprylate/caprate, isopropyl isostearate, cyclomethicone, butylene glycol dicaprylate/dicaprate, dicaprylyl carbonate, isopropyl stearate, dibutyl adipate, isodecyl neopentanoate, isohexadecane, C13-16 isoparaffin and/or dicaprylyl ether,
wherein the proportion of this oil or these oils is selected in the range of 10 to 20% by weight, based on the total mass of the preparation,
wherein to measure the spreadability value (in mm²/10 min), 20 µl of the substance to be investigated are dripped centrally onto a red band filter paper from Schleicher & Schüll, a stopwatch is immediately started and, after 10 minutes, the area which has been wetted by the substance in this time in a room at constant temperature at 25°C ± 1°C is measured,
wherein the proportion of polyethylene glycols and/or polyethylene glycol derivatives is below 1% by weight, especially 0% by weight, based on the total mass of the preparation,
and wherein
one or more powder raw materials are present.

2. Preparation according to Claim 1, **characterized in that** only two W/O emulsifiers are present.

3. Preparation according to either of the preceding claims, **characterized in that** the oils with a spreadability value greater than 600 mm²/10 min have a viscosity of 10 mPas and less.

4. Preparation according to any of the preceding claims, **characterized in that** the proportion of oils with a spreadability value greater than 600 mm²/10 min is selected in the range of 30 to 90% by weight, preferably 40 to 80, particularly preferably 40 to 60% by weight, based on the total mass of the oil phase.

5. Preparation according to any of the preceding claims, **characterized in that** the oils with a spreadability value greater than 600 mm²/10 min are selected from the group of isopropyl palmitate and C13-16 isoparaffin.

6. Preparation according to any of the preceding claims, **characterized in that** one oil with a spreadability value greater than 600 mm²/10 min and at least one oil with a spreadability value greater than 1000 mm²/10 min are present.

7. Preparation according to any of the preceding claims, comprising at least one skin moisturizer at a proportion of in total 5 to 20% by weight, based on the total mass of the preparation.

8. Preparation according to Claim 7, **characterized in that** the skin moisturizer selected is glycerin.

9. Preparation according to Claim 1, **characterized in that** the proportion of powder raw materials is up to 5% by weight, in particular 0.2 to 2% by weight, based on the total mass of the preparation.

10. Preparation according to Claim 1 or 9, **characterized in that** the powder raw material selected is aluminium starch octenylsuccinate and/or talc.

11. Preparation according to any of the preceding claims, **characterized in that** the proportion of water is selected in the range of 40 to 80% by weight, in particular between 55 and 65% by weight, based on the total mass of the preparation.

## Revendications

1. Préparation cosmétique et/ou dermatologique fluide à température ambiante à base d'une émulsion eau dans huile comprenant
au moins deux émulsifiants E/H, **caractérisée en ce qu'**un dilinoléate dimère de polyglycéryle-3 de diisostéaroyle et un diisostéarate/polyhydroxystéarate/sébaçate de polyglycéryle-4 sont choisis en tant qu'émulsifiants E/H et
au moins une huile dotée d'une valeur de dispersion supérieure à 600 mm²/10 min, choisie dans le groupe du palmitate d'isopropyle, du myristate d'isopropyle, du caprylate/caprate de coco, de l'isostéarate d'isopropyle, d'une cyclométhicone, du dicaprylate/dicaprate de butylèneglycol, du carbonate de dicaprylyle, du stéarate d'isopropyle, de l'adipate de dibutyle, du néopentanoate d'isodécyle, de l'isohexadécane, d'une isoparaffine en C13 à 16 et/ou de l'éther de dicaprylyle,
la teneur de cette huile ou de ces huiles étant choisie dans la plage de 10 à 20 % en poids, par rapport à la masse totale de la préparation,
dans laquelle, pour la mesure de la valeur de dispersion (en mm²/10 min), une goutte de 20 µl de la substance devant être testée est placée au centre d'un papier filtre de ruban rouge de la société Schleicher & Schüll, un chronomètre est en même temps déclenché et après 10 minutes, la surface qui a été mouillée pendant ce temps par la substance est mesurée, la mesure étant réalisée dans un espace à température constante de 25 °C ± 1 °C,
la teneur en polyéthylèneglycols et/ou en dérivés de polyéthylèneglycol étant inférieure à 1 % en poids, en particulier étant de 0 % en poids, par rapport à la masse totale de la préparation, et
une ou plusieurs matières premières en poudre étant contenues.

2. Préparation selon la revendication 1, **caractérisée en ce que** seulement deux émulsifiants E/H sont contenus.

3. Préparation selon l'une quelconque des revendications précédentes, **caractérisée en ce que** les huiles dotées d'une valeur de dispersion supérieure à 600 mm²/10 min présentent une viscosité de 10 mPas et moins.

4. Préparation selon l'une quelconque des revendications précédentes, **caractérisée en ce que** la teneur en huiles dotées d'une valeur de dispersion supérieure à 600 mm²/10 min. est choisie dans la plage de 30 à 90 % en poids, préférablement de 40 à 80, particulièrement préférablement de 40 à 60 % en poids, par rapport à la masse totale de la phase huileuse.

5. Préparation selon l'une quelconque des revendications précédentes, **caractérisée en ce que** les huiles dotées d'une valeur de dispersion supérieure à 600 mm²/10 min. sont choisies dans le groupe du palmitate d'isopropyle et d'une isoparaffine en C13 à 16.

6. Préparation selon l'une quelconque des revendications précédentes, **caractérisée en ce qu'**une huile dotée d'une valeur de dispersion supérieure à 600 mm²/10 min. et au moins une huile dotée d'une valeur de dispersion supérieure à 1 000 mm²/10 min. sont contenues.

7. Préparation selon l'une quelconque des revendications précédentes comprenant au moins un agent hydratant pour la peau à raison d'une teneur totale de 5 à 20 % en poids, par rapport à la masse totale de la préparation.

8. Préparation selon la revendication 7, **caractérisée en ce que** de la glycérine est utilisée en tant qu'agent hydratant pour la peau.

9. Préparation selon la revendication 1, **caractérisée en ce que** la proportion de matières premières en poudre est de jusqu'à 5 % en poids, en particulier de 0,2 à 2 % en poids, par rapport à la masse totale de la préparation.

10. Préparation selon la revendication 1 ou 9, **caractérisée en ce que** de l'octénylsuccinate d'amidon d'aluminium et/ou du talc est choisi en tant que matière première en poudre.

11. Préparation selon l'une quelconque des revendications précédentes, **caractérisée en ce que** la teneur en eau est choisie dans la plage de 40 à 80 % en poids, en particulier entre 55 et 65 % en poids, par rapport à la masse totale de la préparation.
